# EUROPÄISCHE PATENTSCHRIFT

(11) **EP 3 292 845 B1**
(45) Veröffentlichungstag und Bekanntmachung des Hinweises auf die Patenterteilung: **14.08.2019**
(21) Anmeldenummer: 16187791.5
(22) Anmeldetag: 08.09.2016
(51) Int. Cl.: A61F 13/00, A61F 13/02, A61F 13/84

(54) **INDIKATORAUFLAGE UND VERFAHREN ZUM ANZEIGEN EINER VERKEIMUNG BEI EINER WUNDE**
INDICATOR OVERLAY AND METHOD FOR DISPLAYING CONTAMINATION OF A WOUND
SUPPORT D'INDICATEURS ET PROCÉDÉ D'AFFICHAGE D'UNE CONTAMINATION DE PLAIE

(43) Veröffentlichungstag der Anmeldung: 14.03.2018
(73) Patentinhaber: Sefar AG, 9410 Heiden (CH)
(72) Erfinder: GERDES, Gerd, 9030 Abtwil (CH); WEBER, Jérémie, 6833 Klaus (AT); PREYSCH, Marc, 8590 Romanshorn (CH)
(74) Vertreter: Wunderlich & Heim Patentanwälte Partnerschaftsgesellschaft mbB

(56) Entgegenhaltungen:
- WO-A1-99/36017
- WO-A1-2011/080427
- WO-A1-2013/147662
- DE-A1-102010 001 855
- US-A- 5 181 905
- US-A1- 2013 300 100

## Beschreibung

Die Erfindung betrifft eine Indikatorauflage mit einem Trägerelement, welches zumindest bereichsweise mit einem Markermaterial versehen ist, welches bei einer bestimmten mikrobiologischen Verkeimung eine Farbe ändert, gemäß dem Oberbegriff des Anspruchs 1.

Die Erfindung betrifft weiterhin ein Verfahren zum Anzeigen einer Verkeimung bei Gegenständen gemäß dem Oberbegriff des Anspruchs 12.

Aus der WO 2000/024438 A1 ist eine Zusammensetzung bekannt, welche eine mikrobiologische Verkeimung an medizinischen Geräten und Materialien anzeigen kann. Dieser Indikator umfasst einen Stoff oder eine Stoffzusammensetzung, welche abhängig von einem Vorhandensein eines mikrobiologischen Milieus zu einem Farbumschlag führen kann. Hierdurch wird medizinischem Personal signalisiert, dass eine Verkeimung vorhanden ist, oder zumindest ein bestimmtes Niveau erreicht ist.

Weiterhin ist es bekannt, derartige Indikatorstoffe auch bei Wundauflagen vorzusehen. Hierdurch kann medizinischem Personal frühzeitig ein Hinweis zu einem Wundverlauf gegeben werden. Insbesondere kann durch einen entsprechenden Indikator angezeigt werden, dass ein Wechsel der Wundauflage aufgrund des Erreichens eines bestimmten Verkeimungsniveaus sinnvoll oder notwendig ist. Weitergehende Informationen können einer derartigen Wundauflage nicht entnommen werden.

Eine gattungsbildende Wundauflage mit mehreren Markermaterialien, welche bei unterschiedlichen Verkeimungen ansprechen, ist aus der WO 2011/080427 A1 bekannt. Die Markermaterialien sind feldartig angeordnet.

Aus der DE 10 2010 001 855 A1 und der WO 2013/147662 A1 gehen weitere Wundauflagen mit feldartig angeordneten Markermaterialien hervor.

Die US 5,181,905 offenbart ein Pflaster mit Feldern von Markermaterialien, wobei neben den Feldern jeweils eine Zahl oder ein Buchstabe aufgedruckt ist.

Der Erfindung liegt die **Aufgabe** zugrunde, eine Indikatorauflage und ein Verfahren zum Anzeigen einer Verkeimung anzugeben, mit welchen eine umfassendere Information zur Verkeimung ermöglicht werden.

Die Aufgabe wird zum einen durch eine Indikatorauflage mit den Merkmalen des Anspruchs 1 und zum anderen durch ein Verfahren mit den Merkmalen des Anspruchs 12 gelöst. Bevorzugte Ausführungsformen der Erfindung sind in den jeweils abhängigen Ansprüchen angegeben.

Die erfindungsgemäße Indikatorauflage ist unter anderem dadurch gekennzeichnet, dass ein erstes Markermaterial vorgesehen ist, welches bei einer ersten bestimmten mikrobiologischen Verkeimung die Farbe ändert, und dass mindestens ein zweites Markermaterial vorgesehen ist, welches die Farbe bei einer zweiten bestimmten mikrobiologischen Verkeimung ändert, welches sich von der ersten mikrobiologischen Verkeimung unterscheidet.

Eine Grundidee der Erfindung besteht darin, die Indikatorauflage mit mindestens zwei unterschiedlichen Markermaterialien zu versehen, welche bei unterschiedlichen Zuständen oder Arten einer mikrobiologischen Verkeimung ansprechen. Beispielsweise kann ein erstes Markermaterial eine leichte Verkeimung durch Verfärbung anzeigen, während das andere Markermaterial erst bei einer stärkeren Verkeimung anspricht und sich verfärbt. Dies kann für medizinisches Personal die zusätzliche Information geben, dass sich beispielsweise die Entzündung einer Wunde verstärkt hat. Alternativ oder ergänzend kann es auch vorgesehen sein, dass die unterschiedlichen Markermaterialien auf unterschiedliche Arten von Keimen ansprechen, so dass durch die Indikatorauflage eine Information zur Art der Verkeimung angezeigt werden kann. Je größer die Anzahl der Markermaterialien, um so differenzierter und genauer kann eine Verkeimung angezeigt werden.

Die erfindungsgemäße Indikatorauflage wird vorzugsweise an Wunden eines menschlichen oder tierischen Patienten eingesetzt. Die Indikatorauflage kann aber auch zu anderen Einsatzzwecken verwendet werden, etwa bei medizinischen Geräten oder Instrumenten, zu welchen eine Information über einen Grad oder eine Art der Verkeimung für deren Gebrauch wesentlich ist.

Grundsätzlich können die unterschiedlichen Markermaterialien, welche in einer Anzahl von zwei oder mehr vorgesehen sind, gleichmäßig oder vollflächig über die Indikatorauflage verteilt angeordnet sein. In diesem Fall bewirken die unterschiedlichen Markermaterialien unterschiedliche Farbumschläge, so dass die angezeigte Farbe eine Information, insbesondere auf die Art oder den Umfang der Verkeimung gibt. Besonders bevorzugt ist es nach einer Ausführungsform der Erfindung, dass das erste Markenmaterial auf dem Trägerelement beabstandet zu dem mindestens zweiten Markermaterial angeordnet ist. Somit überdecken sich die Markermaterialien nicht und können eine eindeutige Anzeige bieten. Die Markermaterialien können ein- oder zweiseitig auf dem flächigen Trägerelement aufgebracht sein.

Die Markermaterialien sind vorzugsweise physikalisch getrennt, etwa auf separaten Grundträgern vorgesehen, welche auf dem Trägerelement angeordnet oder eingesetzt sind. Die Grundträger können eigenständige Indikatorelemente sein, welche sich in Material, Form, Größer, Farbe etc. unterscheiden. Diese Indikatorelemente sind mit den Markermaterialien versehen und vorzugsweise über Trennelemente getrennt auf dem Trägerelement angeordnet oder angebracht.

Nach einer Weiterbildung der erfindungsgemäßen Indikatorauflage ist es vorteilhaft, dass das Trägerelement eine Auflageseite zum Auflegen insbesondere auf eine Wunde und eine Oberseite aufweist, welche von der Auflageseite abgewandt ist, und dass eine Farbe der Markermaterialien auf der Oberseite erkennbar ist. Das Trägerelement kann dabei ein- oder mehrschichtig aufgebaut sein. Die Markermaterialien befinden sich dabei vorzugsweise in einem oberen Bereich des Trägerelementes, etwa einem Deckelement, wobei die Markermaterialien in Verbindung mit der verkeimten Fläche sind, auf welche die Indikatorauflage aufgebracht wird. Die Anordnung der Markermaterialien ist dabei so ausgewählt, dass ein Farbumschlag zuverlässig an der Oberseite erkennbar ist.

Nach einer Weiterbildung der Erfindung besteht eine vorteilhafte Ausführungsform darin, dass das Trägerelement an der Oberseite eine transparente Schutzschicht aufweist. Durch die transparente Schicht wird eine äußere Schutzschicht für die Markermaterialien gebildet. Dies erhöht die Langlebigkeit und Funktionsfähigkeit der Indikatorauflage. Besonders vorteilhaft ist es nach der Erfindung, dass das Trägerelement mindestens ein Monofilamentgewebe aufweist. Dieses ist biokompatibel und ist somit besonders schonend bei der Verwendung der Indikatorauflage als einer Wundauflage.

Die Auflageseite ist durch ein biokompatibles Monofilamentgewebe aus Monofilamentfäden, vorzugsweise mit einer glatten Oberfläche gebildet. Auf dem Monofilamentgewebe ist an einer von der Auflage beziehungsweise der Wunde abgewandten Oberseite ein Deckelement aufgebracht, welches mit dem Markermaterial versehen ist.

Das Deckelement mit dem Markermaterial steht somit nicht unmittelbar mit dem verletzten Gewebe der Wunde in Kontakt. Vielmehr ist zwischen der Wunde und dem Deckelement mit den Markermaterialien eine Trennschicht vorgesehen, welche vorzugsweise aus einem biokompatiblen Monofilamentgewebe gebildet ist. Das Monofilamentgewebe ist aus feinen drahtähnlichen Monofilamentgarnen gebildet. Vorzugsweise ist das Deckelement mit den Markermaterialien so ausgebildet, dass dieses erst bei einer bestimmten Verkeimung hinter dem Monofilamentgewebe als Trennschicht anspricht. Das Deckelement kann die Markermaterialien in fester, flüssiger und/oder pastöser Form aufnehmen.

Zudem wird durch die Trennschicht einer Irritation der Wunde durch die vorgesehenen Markermaterialien entgegengewirkt. Derartige Wechselwirkungen können zu einer Störung der Wundheilung führen.

Das Trägerelement ist vorzugsweise aus einem Gewebe gebildet, welches nicht irritierend ist, insbesondere nicht zytotoxisch, pyrogenfrei und hypoallergen ist.

Vorzugsweise ist das biokompatible Monofilamentgewebe so stabil ausgebildet, dass es als eine Trägerschicht dient, auf welcher mindestens ein Deckelement fest angebracht ist. Dieses Deckelement kann dabei selbst eine einzelne Schicht oder mehrere Schichten aufweisen. Entsprechend dem Einsatzzweck der Indikatorauflage kann das Deckelement zusätzlich in einer gewünschten Weise, etwa zum Aufnehmen von Wundsekret oder zum Aufbringen von wundheilungsfördernden Mitteln, etwa einer Salbe oder Flüssigkeit, ausgebildet sein. Das Deckelement kann ein Gewebe, ein

Vlies und/oder ein sonstiges Material aufweisen, welches zum Aufnehmen der Markermaterialien fähig ist.

Eine bevorzugte Ausführungsform der Erfindung besteht darin, dass das Monofilamentgewebe als ein Einfachgewebe mit offenmaschiger Struktur ausgebildet ist, welches eine Porengröße von 5 µm bis 500 µm, vorzugsweise 20 µm bis 300 µm aufweist, und dass die Poren einen Anteil von 15% bis 70% der Fläche des Monofilamentgewebes bilden. Das erfindungsgemäße Monofilamentgewebe ist gemäß dieser Ausführungsform also besonders feinporig, so dass zwar einerseits ein Luft- und Flüssigkeitsdurchtritt durch die Poren möglich ist, jedoch andererseits ein Verwachsen oder Umgreifen des Monofilamentgewebes durch Gewebematerial kaum möglich ist. Es wird so eine schützende, aber durchlässige Indikatorauflage, insbesondere Wundauflage geschaffen, welche jedoch weiterhin eine klare Abgrenzung und damit eine leichte Lösbarkeit von einer Wunde sicherstellt. Die gute Durchlässigkeit für Luft und Flüssigkeit ist insbesondere dadurch gegeben, dass die Poren einen Anteil von 15% bis 70% der Fläche des Monofilamentgewebes, vorzugsweise 40% bis 70% bilden. Auf diese Weise wird eine hohe Durchlässigkeit erreicht.

Vorzugsweise ist das Trägerelement aus Fäden aus PA, PET, PP, PVDF und/oder PTFE gebildet. Die Fäden, insbesondere Monofilamentfäden, weisen eine Durchmessergröße zwischen 20 µm bis 500 µm, vorzugsweise zwischen 30 µm bis 150 µm auf. In einer einfachen Ausführungsform ist die Indikatorauflage mit zwei unterschiedlichen Markermaterialien versehen. Eine besonders gute Information wird nach einer weiteren Ausführungsvariante der Erfindung dadurch erreicht, dass mehr als zwei verschiedene Markermaterialien vorgesehen sind.

Unter unterschiedlichen Markermaterialien ist zu verstehen, dass diese stofflich unterschiedlich sind oder in einer unterschiedlichen Konzentration oder in einer sonstigen Weise derart unterschiedlich ausgebildet oder angeordnet sind, dass diese bei unterschiedlichen Arten und/oder Graden von Verkeimungen ansprechen.

Eine besonders gute Anzeige unterschiedlicher Verkeimungen wird nach der Erfindung dadurch erreicht, dass die Markermaterialien in Form von Buchstaben, Zahlen, Mustern und/oder Symbolen aufgebracht sind. Insbesondere können die Buchstaben, Zahlen, Muster und/oder Symbole so gewählt sein, dass diese eine unmittelbare Information zur Stärke oder Art der Verkeimung mitteilen. Als Muster können etwa Linien- oder Punktmuster vorgesehen sein. Auch können etwa die Bezeichnungen der Infektionskeime mit dem Markermaterial vollständig oder in Abkürzung geschrieben sein. So kann etwa bei einer Verkeimung durch Kolibakterien das Wort "Koli" erscheinen oder ein Symbol auf das vorgedruckte Wort verweisen.

Bei der Verwendung derartiger Zeichen können die Markermaterialien in die gleiche Farbe umschlagen. Besonders bevorzugt ist jedoch, wenn die unterschiedlichen Markermaterialien auch zu einer unterschiedlichen Verfärbung führen.

Die Markermaterialien können grundsätzlich auf beliebige Weise auf das Trägerelement oder auf eine Schicht des Trägerelementes aufgebracht sein. Besonders bevorzugt ist es nach einer Ausführungsvariante der Erfindung, dass die Markermaterialien durch Siebdruck, Sprühdruck oder Stempeln aufgebracht sind. Diese eignen sich auch insbesondere zum Aufbringen von flüssigen oder pastösen Markermaterialien in Form von Buchstaben, Zahlen, Mustern und/oder Symbolen.

Eine weitere bevorzugte Ausführungsform der Erfindung besteht darin, dass an einer Auflageseite des Trägerelementes zumindest bereichsweise eine Haftschicht vorgesehen ist. Die Haftschicht ist dabei üblicherweise ein Klebstoffauftrag, welcher zum Aufbringen insbesondere auf Haut geeignet ist. Die Haftschicht kann dabei unmittelbar auf der Auflageseite aufgebracht werden.

Grundsätzlich kann als Markermaterial, welches als ein Indikator zum Anzeigen eines Verkeimungszustands, insbesondere eines mikrobiologischen Wundmilieus und insbesondere einer Verkeimung einer Wunde dient, jedes geeignete Material verwendet werden. Besonders bevorzugt ist es nach einer Weiterbildung der Erfindung, dass das Markermaterial zumindest einen Farbstoff, insbesondere einen reaktiven Farbstoff, einen fluoreszierenden Farbstoff, einen pH-empfindlichen Stoff, Enzyme, Zellen, ein Hydrogel, einen temperaturempfindlichen Stoff, ein Salz, eine Salbe und Alginate umfasst. Diese Stoffe sind dabei so ausgewählt, dass sie bei einem entsprechenden Verkeimungsmilieu etwa durch Abbauprodukte der Bakterien zu einem Farbumschlag führen.

Weiterhin ist es nach einer vorteilhaften Ausführungsvariante der Erfindung vorgesehen, dass die Markermaterialien in einem Ausgangszustand farblos sind oder die gleiche Farbe aufweisen, wobei sich eine Verfärbung bei Erreichen der jeweils bestimmten Verkeimung einstellt. Insbesondere können derartige Markermaterialien bei herkömmlichen Wundauflagen eingesetzt werden, so dass diese als nicht störend empfunden werden. Das Trägerelement hat vorzugsweise eine neutrale Farbe, etwa weiß, während die Markermaterialien sich zu einer Kontrastfarbe, etwa rot, blau, grün etc., ändern.

Das erfindungsgemäße Verfahren ist dadurch gekennzeichnet, dass eine erfindungsgemäße Indikatorauflage aufgelegt wird, dass bei einer ersten mikrobiologischen Verkeimung sich die Farbe eines ersten Markermaterials ändert, und dass bei einer zweiten mikrobiologischen Verkeimung sich die Farbe eines zweiten Markermaterials ändert, wobei sich die erste mikrobiologische Verkeimung von der zweiten mikrobiologischen Verkeimung unterscheidet.

Das erfindungsgemäße Verfahren wird bei Gegenständen, etwa medizinischen Geräten und Instrumenten, eingesetzt. Es ergeben sich die zuvor beschriebenen Vorteile beim Einsatz einer erfindungsgemäßen Indikatorauflage.

Gemäß einer Weiterbildung des Verfahrens nach der Erfindung ist es vorgesehen, dass die unterschiedlichen Markermaterialien auf einen unterschiedlichen Grad der mikrobiologischen Verkeimung und/oder bei unterschiedlichen mikrobiologischen Keimen ansprechen. Insbesondere können die Markermaterialien so ausgewählt sein, dass diese jeweils bei unterschiedlichen Keimen ansprechen.

Eine besonders gute Verkeimungsanzeige wird nach einer Weiterbildung des erfindungsgemäßen Verfahrens dadurch erzielt, dass die Markermaterialien in einem Ausgangszustand farblos sind oder die gleiche Farbe aufweisen und sich bei Erreichen der jeweiligen bestimmten mikrobiologischen Verkeimung verfärben.

Weiterhin ist es vorteilhaft, dass das erste Markermaterial und das mindestens zweite Markermaterial bei Erreichen der bestimmten mikrobiologischen Verkeimung eine erste Farbe beziehungsweise mindestens eine zweite Farbe annehmen, welche unterschiedlich sind. Durch das Umschlagen zu unterschiedlichen Farben wird es für medizinisches Personal besonders gut verdeutlicht, welche Verkeimung vorliegt.

Nachfolgend wird die Erfindung anhand eines bevorzugten Ausführungsbeispiels weiter erläutert, welches schematisch in den Zeichnungen dargestellt ist. In den Zeichnungen zeigen:
Fig. 1 eine schematische Draufsicht auf eine erfindungsgemäße Indikatorauflage; und
Fig. 2 eine schematische, nicht maßstabsgetreue Querschnittsansicht der Indikatorauflage von Fig. 1.

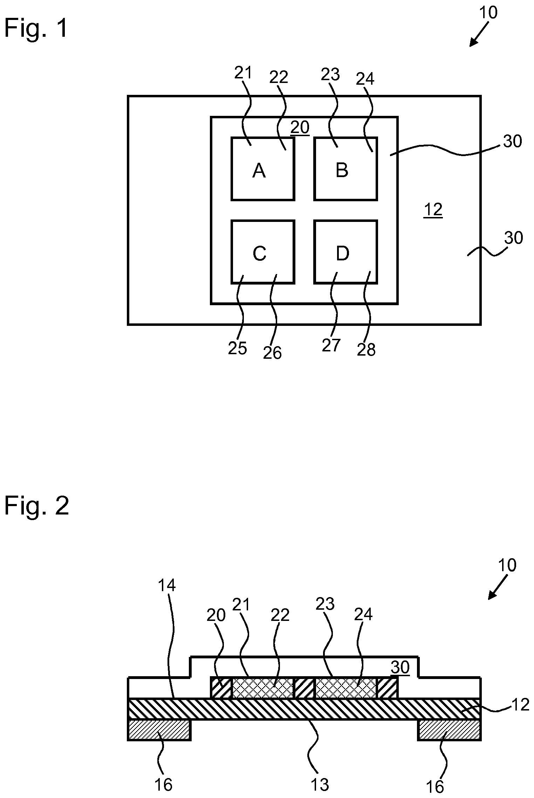

In den Figuren 1 und 2 ist eine erfindungsgemäße Indikatorauflage 10 dargestellt, welche ein rechteckiges Trägerelement 12 aufweist. Das Trägerelement 12 ist flexibel und als ein biokompatibles Monofilamentgewebe ausgebildet. An einer Auflageseite 13, welche zum Auflegen auf eine Wunde vorgesehen ist, sind seitlich zwei streifenförmige Haftschichten 16 vorgesehen. Eine einzelne Haftschicht 16 besteht dabei aus einem Klebstoffauftrag, mit welcher die Indikatorauflage etwa entlang des Randes einer Wunde lösbar befestigt werden kann.

Das flexible Trägerelement 12 ist mit Poren oder Öffnungen versehen, so dass eine Verbindung des Wundmilieus an der Auflageseite 13 zu einem flächigen Deckelement 20 besteht, welches an einer Oberseite 14 des Trägerelementes 12 aufgebracht ist. Das Deckelement 20 kann ebenfalls als ein Gewebe, insbesondere ein Monofilamentgewebe, oder ein Vlies oder ein anderes Beschichtungselement ausgebildet sein, welches zur Aufnahme von Markermaterialien geeignet ist.

Bei dem dargestellten Ausführungsbeispiel sind in dem Deckelement 20 ein erster Bereich 21, ein zweiter Bereich 23, ein dritter Bereich 25 und ein vierter Bereich 27 räumlich voneinander beabstandet angeordnet. Die Bereiche 21, 23, 25, 27 sind mit unterschiedlichen Markermaterialien, nämlich einem ersten Markermaterial 22, einem zweiten Markermaterial 24, einem dritten Markermaterial 26 beziehungsweise einem vierten Markermaterial 28 versehen. Die Markermaterialien 22, 24, 26, 28 sind in dem dargestellten Beispiel als Flüssigkeit aufgebracht und in das Deckelement 20 eingedrungen. Dabei sind die Markermaterialien 22, 24, 26, 28 jedoch in den entsprechenden Bereichen 21, 23, 25, 27 räumlich beabstandet angeordnet. In die Zwischenräume kann ein Trennmittel, etwa ein Lack, vorgesehen sein, um die einzelnen Bereiche 21, 23, 25, 27 zuverlässig voneinander abzugrenzen.

Die Markermaterialien 22, 24, 26, 28 sind dabei unterschiedlich ausgewählt, so dass diese bei einer jeweils vorgegebenen unterschiedlichen Verkeimung zu einem Farbumschlag führen. Vorzugsweise ergibt sich jeweils eine Änderung zu einer unterschiedlichen Farbe. Zur weiteren verbesserten Signalisierung einer Verkeimung ist bei der dargestellten Indikatorauflage 10 in jedem Bereich 21, 23, 25, 27 eine Beschriftung vorgesehen, welche schematisch durch die Buchstaben A, B, C beziehungsweise D dargestellt ist. Die Beschriftung kann auch unmittelbar die Art der Infektionskeime bezeichnen. Ergibt sich ein Farbumschlag im ersten Bereich 21 mit dem ersten Markermaterial 22, wird dem medizinischen Personal bei einem Farbumschlag in dem ersten Bereich 21 signalisiert, dass eine Infektion mit dem Keim A gegeben ist.

Das Trägerelement 12 mit dem Deckelement 20 ist an der Oberseite 14 mit einer transparenten Schutzschicht 30 versehen. Diese schützt das Deckelement mit den Markermaterialien 22, 24, 26, 28 vor äußeren Einflüssen, erlaubt jedoch weiterhin das Erkennen eines Farbumschlages in den Bereichen 21, 23, 25 oder 28.

## Patentansprüche

1. Indikatorauflage, insbesondere zum Auflegen auf eine Wunde, mit einem Trägerelement (12), welches zumindest bereichsweise mit Markermaterialien versehen ist, welche bei einer bestimmten mikrobiologischen Verkeimung ihre Farbe ändern,
**dadurch gekennzeichnet,**
- **dass** eine Auflageseite des Trägerelementes (12) beim Auflegen auf die Wunde durch ein biokompatibles Monofilamentgewebe aus Monofilamentfäden gebildet ist,
- **dass** die Markermaterialien (22, 24, 26, 28) in Form von Buchstaben und/oder Zahlen aufgebracht sind,
- **dass** ein erstes Markermaterial (22) vorgesehen ist, welches bei einer ersten bestimmten mikrobiologischen Verkeimung die Farbe ändert, und
- **dass** mindestens ein zweites Markermaterial (24, 26, 28) vorgesehen ist, welches die Farbe bei einer zweiten bestimmten mikrobiologischen Verkeimung ändert, welche sich von der ersten mikrobiologischen Verkeimung hinsichtlich Art oder Umfang der Verkeimung unterscheidet.

2. Indikatorauflage nach Anspruch 1,
**dadurch gekennzeichnet,**
**dass** das erste Markermaterial (22) auf dem Trägerelement (12) beabstandet zu dem mindestens zweiten Markermaterial (24, 26, 28) angeordnet ist.

3. Indikatorauflage nach Anspruch 1 oder 2,
**dadurch gekennzeichnet,**
**dass** das Trägerelement (12) eine Auflageseite (13) zum Auflegen insbesondere auf eine Wunde und eine Oberseite (14) aufweist, welche von der Auflageseite (13) abgewandt ist, und
**dass** eine Farbe der Markermaterialien (22, 24, 26, 28) auf der Oberseite (14) erkennbar ist.

4. Indikatorauflage nach Anspruch 3,
**dadurch gekennzeichnet,**
**dass** das Trägerelement (12) an der Oberseite (14) eine transparente Schutzschicht (30) aufweist.

5. Indikatorauflage nach einem der Ansprüche 1 bis 4,
**dadurch gekennzeichnet,**
**dass** mehr als zwei verschiedene Markermaterialien (22, 24, 26, 28) vorgesehen sind.

6. Indikatorauflage nach einem der Ansprüche 1 bis 5,
**dadurch gekennzeichnet,**
**dass** die Buchstaben und/oder Zahlen, in deren Form die Markermaterialien (22, 24, 26, 28) aufgebracht sind, eine unmittelbare Information zur Stärke oder Art der Verkeimung angeben.

7. Indikatorauflage nach einem der Ansprüche 1 bis 6,
**dadurch gekennzeichnet,**
**dass** die Markermaterialien (22, 24, 26, 28) durch Siebdruck, Sprühdruck oder Stempeln aufgebracht sind.

8. Indikatorauflage nach einem der Ansprüche 1 bis 7,
**dadurch gekennzeichnet,**
**dass** die Monofilamentfäden des Monofilamentgewebes des Trägerelementes (12) mit einer glatten Oberfläche ausgebildet sind.

9. Indikatorauflage nach einem der Ansprüche 1 bis 8,
**dadurch gekennzeichnet,**
**dass** an einer Auflageseite (13) des Trägerelementes (12) zumindest bereichsweise eine Haftschicht (16) vorgesehen ist.

10. Indikatorauflage nach einem der Ansprüche 1 bis 9,
**dadurch gekennzeichnet,**
**dass** das Markermaterial (22, 24, 26, 28) zumindest einen Farbstoff, insbesondere einen reaktiven Farbstoff, einen fluoreszierenden Farbstoff, einen pH-empfindlichen Stoff, Enzyme, Zellen, ein Hydrogel, einen temperaturempfindlichen Stoff, ein Salz, eine Salbe und/oder Alginate umfasst.

11. Indikatorauflage nach einem der Ansprüche 1 bis 10,
**dadurch gekennzeichnet,**
**dass** die Markermaterialien (22, 24, 26, 28) in einem Ausgangszustand farblos sind oder die gleiche Farbe aufweisen, wobei sich eine Verfärbung bei Erreichen der jeweils bestimmten Verkeimung einstellt.

12. Verfahren zum Anzeigen einer Verkeimung bei Gegenständen, insbesondere medizinischen Geräten oder Instrumenten, wobei eine Indikatorauflage (10) mit verschiedenen Markermaterialien aufgelegt wird, wobei bei einer bestimmten mikrobiologischen Verkeimung sich die Farbe der Markermaterialien ändert,
**dadurch gekennzeichnet,**
- **dass** eine Indikatorauflage (10) nach einem der Ansprüche 1 bis 11 aufgelegt wird,
- **dass** bei einer ersten mikrobiologischen Verkeimung sich die Farbe eines ersten Markermaterials (22) ändert, und
- **dass** bei einer zweiten mikrobiologischen Verkeimung sich die Farbe eines zweiten Markermaterials (24) ändert, wobei sich die erste mikrobiologische Verkeimung von der zweiten mikrobiologischen Verkeimung unterscheidet,
- wobei die veränderten Markermaterialien Buchstaben und/oder Zahlen auf dem Trägerelement (12) anzeigen.

13. Verfahren nach Anspruch 12,
**dadurch gekennzeichnet,**
**dass** die unterschiedlichen Markermaterialien (22, 24, 26, 28) auf einen unterschiedlichen Grad der mikrobiologischen Verkeimung und/oder bei unterschiedlichen mikrobiologischen Keimen ansprechen.

14. Verfahren nach Anspruch 12 oder 13,
**dadurch gekennzeichnet,**
**dass** die Markermaterialien (22, 24, 26, 28) in einem Ausgangszustand farblos sind oder die gleiche Farbe aufweisen und sich bei Erreichen der jeweiligen bestimmten mikrobiologischen Verkeimung verfärben.

15. Verfahren nach einem der Ansprüche 12 bis 14,
**dadurch gekennzeichnet,**
**dass** das erste Markermaterial (22) und das mindestens zweite Markermaterial (22, 24, 26, 28) bei Erreichen der bestimmten mikrobiologischen Verkeimung eine erste Farbe beziehungsweise mindestens eine zweite Farbe annehmen, welche unterschiedlich sind.

## Claims

1. Indicator dressing, in particular for placing upon a wound, with a carrier element (12), which is provided, at least in regions, with marker materials, which, in a specifically microbiological contamination, changes its color,
**characterized in that**
- a placement side of the carrier element (12) is designed by a biocompatible monofilament fabric made of monofilament threads during placing upon the wound.
- **in that** the marker materials (22, 24, 26, 28) are applied in the form of letters and/or numbers.
- **in that** a first marker material (22) is provided, which, in a first specifically microbiological contamination, changes the color, and
- **in that** at least a second marker material (24, 26, 28) is provided, which changes the color in a second specifically microbiological contamination, which differs from the first microbiological contamination in regard of type and amount of the contamination.

2. Indicator dressing according to claim 1,
**characterized in that**
the first marker material (22) is arranged on the carrier element (12) distanced to the at least second marker material (24, 26, 28).

3. Indicator dressing according to claim 1 or 2,
**characterized in that**
the carrier element (12) comprises a placement side (13) for placing, in particular on a wound, and an upper side (14) which faces away from the placement side (13), and
**in that** a color of the marker materials (22, 24, 26, 28) can be discerned on the upper side (14).

4. Indicator dressing according to claim 3,
**characterized in that**
the carrier element (12) comprises, on the upper side (14), a transparent protective layer (30).

5. Indicator dressing according to one of the claims 1 to 4,
**characterized in that**
more than two different marker materials (22, 24, 26, 28) are provided.

6. Indicator dressing according to one of the claims 1 to 5,
**characterized in that**
the letters and/or numbers in the form of which the marker materials (22, 24, 26, 28) are applied, indicate a direct information to the intensity or type of the contamination.

7. Indicator dressing according to one of the claims 1 to 6,
**characterized in that**
the marker materials (22, 24, 26, 28) are applied through screen printing, spray printing or stamping.

8. Indicator dressing according to one of the claims 1 to 7,
**characterized in that**
the monofilament threads of the monofilament fabric of the carrier element (12) are designed with a smooth surface.

9. Indicator dressing according to one of the claims 1 to 8,
**characterized in that**
an adhesive layer (16) is provided, at least in regions, on a placement side (13) of the carrier element (12).

10. Indicator dressing according to one of the claims 1 to 9,
**characterized in that**
the marker material (22, 24, 26, 28) includes at least one colorant, in particular a reactive colorant, a fluorescent colorant, a pH-sensitive substance, enzymes, cells, a hydrogel, a temperature-sensitive substance, a salt, an ointment, and/or alginates.

11. Indicator dressing according to one of the claims 1 to 10,
**characterized in that**
the marker materials (22, 24, 26, 28) are colorless in an initial state, or have the same color, wherein a discoloration occurs upon reaching of the respectively specific contamination.

12. Method for indicating a contamination at objects, in particular medical devices or instruments, wherein an indicator dressing (10) with different marker materials is placed-on, wherein the color of the marker materials changes in a specifically microbiological contamination,
**characterized in that**
- an indicator dressing (10) according to one of the claims 1 to 11 is placed-on,
- **in that** in a first microbiological contamination, the color of a first marker material (22) changes, and
- **in that** in a second microbiological contamination, the color of a second marker material (24) changes, wherein the first microbiological contamination differs from the second microbiological contamination.
- wherein the changed marker materials (22, 24, 26, 28) indicate letters and/or numbers on the carrier element (12).

13. Method according to claim 12,
**characterized in that**
the different marker materials (22, 24, 26, 28) react to a different degree of the microbiological contamination and/or in different microbiological germs.

14. Method according to claim 12 or 13,
**characterized in that**
the marker materials (22, 24, 26, 28) are colorless in an initial state, or have the same color and, upon achieving the respective specifically microbiological contamination, discolor.

15. Method according to one of claims 12 to 14,
**characterized in that**
the first marker material (22) and the at least second marker material (22, 24, 26, 28), upon achieving the specifically microbiological contamination, assume a first color or at least one second color, which are different.

## Revendications

1. Support d'indicateur, en particulier destiné à être placé sur une plaie, avec un élément de support (12), qui est pourvu au moins par endroits de matériaux marqueurs, lesquels changent de couleur en cas de contamination microbiologique définie,
**caractérisé en ce**
- **qu'**une face de contact de l'élément de support (12) lors du placement sur la plaie est formée par un tissu à mono filament biocompatible composé de fils mono filaments,
- **que** les matériaux marqueurs (22, 24, 26, 28) sont appliqués sous la forme de lettres et/ou de chiffres,
- **qu'**un premier matériau marqueur (22) est prévu, lequel change de couleur dans le cas d'une première contamination microbiologique définie, et
- **qu'**au moins un deuxième matériau marqueur (24, 26, 28) est prévu, lequel change de couleur dans le cas d'une deuxième contamination microbiologique définie, laquelle diffère de la première contamination microbiologique en termes de type ou d'ampleur de la contamination.

2. Support d'indicateur selon la revendication 1,
**caractérisé en ce**
**que** le premier matériau marqueur (22) est disposé sur l'élément de support (12) de manière espacée par rapport à l'au moins deuxième matériau marqueur (24, 26, 28).

3. Support d'indicateur selon la revendication 1 ou 2,
**caractérisé en ce**
**que** l'élément de support (12) présente une face de contact (13) destinée à être placée en particulier sur une plaie et une face supérieure (14), laquelle est opposée à la face de contact (13), et
**qu'**une couleur des matériaux marqueurs (22, 24, 26, 28) peut être identifiée sur la face supérieure (14).

4. Support d'indicateur selon la revendication 3,
caractérisé e ce
que l'élément de support (12) présente au niveau de la face supérieure (14) une couche de protection (30) transparente.

5. Support d'indicateur selon l'une quelconque des revendications 1 à 4,
**caractérisé en ce**
**que** plus de deux matériaux marqueurs (22, 24, 26, 28) différents sont prévus.

6. Support d'indicateur selon l'une quelconque des revendications 1 à 5,
**caractérisé en ce**
**que** les lettres et/ou les chiffres, sous la forme desquels les matériaux marqueurs (22, 24, 26, 28) sont appliqués, indiquent une information directe portant sur le degré de sévérité ou le type de la contamination.

7. Support d'indicateur selon l'une quelconque des revendications 1 à 6,
**caractérisé en ce**
**que** les matériaux marqueurs (22, 24, 26, 28) sont appliqués par sérigraphie, impression par pulvérisation ou poinçonnage.

8. Support d'indicateur selon l'une quelconque des revendications 1 à 7,
**caractérisé en ce**
**que** les fils mono filaments du tissu à mono filament de l'élément de support (12) sont réalisés avec une surface lisse.

9. Support d'indicateur selon l'une quelconque des revendications 1 à 8,
**caractérisé en ce**
**qu'**au moins par endroits une couche adhésive (16) est prévue au niveau d'une face de contact (13) de l'élément de support (12).

10. Support d'indicateur selon l'une quelconque des revendications 1 à 9,
**caractérisé en ce**
**que** le matériau marqueur (22, 24, 26, 28) comprend au moins un colorant, en particulier un colorant réactif, un colorant fluorescent, une substance sensible au pH, des enzymes, des cellules, un hydrogel, une substance thermo-sensible, un sel, un onguent et/ou des alginates.

11. Support d'indicateur selon l'une quelconque des revendications 1 à 10,
**caractérisé en ce**
**que** les matériaux marqueurs (22, 24, 26, 28) sont incolores dans un état de départ ou présentent la même couleur, dans lequel un changement de couleur apparaît une fois la contamination respectivement définie atteinte.

12. Procédé servant à indiquer une contamination pour des objets, en particulier des appareils ou instruments médicaux, dans lequel un support d'indicateur (10) avec différents matériaux marqueurs est placé, la couleur des matériaux marqueurs étant modifiée dans le cas d'une contamination microbiologique définie,
**caractérisé en ce**
- **qu'**un support d'indicateur (10) selon l'une quelconque des revendications 1 à 11 est placé,
- **que** dans le cas d'une première contamination microbiologique, la couleur d'un premier matériau marqueur (22) change, et
- **que** dans le cas d'une deuxième contamination microbiologique, la couleur d'un deuxième matériau marqueur (24) change, dans lequel la première contamination microbiologique diffère de la deuxième contamination microbiologique,
- dans lequel les matériaux marqueurs modifiés affichent des lettres et/ou des chiffres sur l'élément de support (12).

13. Procédé selon la revendication 12,
**caractérisé en ce**
**que** les différents matériaux marqueurs (22, 24, 26, 28) réagissent à un degré différent de contamination microbiologique et/ou dans le cas de différents germes microbiologiques.

14. Procédé selon la revendication 12 ou 13,
**caractérisé en ce**
**que** les matériaux marqueurs (22, 24, 26, 28) sont incolores dans un état de départ ou présentent la même couleur et changent de couleur une fois la contamination microbiologique définie respective atteinte.

15. Procédé selon l'une quelconque des revendications 12 à 14,
**caractérisé en ce**
**que** le premier matériau marqueur (22) et l'au moins deuxième matériau marqueur (22, 24, 26, 28) adoptent une première couleur ou au moins une deuxième couleur une fois la contamination microbiologique définie atteinte, lesquelles sont différentes.
